# EUROPEAN PATENT SPECIFICATION

(11) **EP 2 359 811 B1**
(45) Date of publication and mention of the grant of the patent: **14.11.2012**
(21) Application number: 11157456.2
(22) Date of filing: 29.08.2006
(51) Int. Cl.: A61K 9/16, A61K 9/50, A61K 9/26

(54) **Dried milled granulate and methods**
Getrocknetes, gemahlenes Granulat und Verfahren
Granulé broyé séché et procédés associés

(30) Priority: 30.08.2005 US 712580 P; 21.08.2006 US 507240
(43) Date of publication of application: 24.08.2011
(62) Divisional of application: 10001313.5
(73) Proprietor: CIMA LABS INC., Eden Prairie, MN 55344-9361 (US)
(72) Inventor: Chastain, Sara, J., Maple Grove, MN 55369 (US); Habib, Walid, Cincinnati, OH 45244 (US); Moe, Derek, Maple Grove, MN 55311 (US)
(74) Representative: Hallybone, Huw George

(56) References cited:
- US-A- 5 178 878
- US-A- 5 618 527
- US-A- 6 039 275

## Description

### BACKGROUND OF THE INVENTION

One traditionally known way of taste masking involves decreasing surface area. This can be accomplished in a number of ways one of which involves increasing overall particle size. There are a number of techniques which are known for increasing surface area and one of those is coating an active pharmaceutical ingredient ("APT") on the surface of a sugar sphere or bead. However, not all materials can be coated in this way, and in some cases, it may not provide appropriate properties.

Another technique which may be used is granulation. Granulation generally encompasses wet or dry techniques, the oldest and perhaps most common of which is wet granulation. In wet granulation, various materials are mixed together with a binder and/or a granulating liquid, one or both of which acts to glue smaller particles together to form agglomerates or granules. Granules can be dried and used to form tablets, capsules or other dosage forms.

To some degree, the degree of increase in particle size and the resulting particle size distribution of the wet granulate depends on the type of device being used and its settings (such as, for example, impeller speed and chopper speed), the volume of material used, the relative portion of wet and dry ingredients and their character, the amount of time the material is worked in the granulator and the like. Of course, depending on the foregoing, it is also possible that the resulting wet granulate could have either the same or an even smaller average particle size. However, generally, wet granulation is a technique meant to increase particle size and, in the context of taste masking, to decrease surface area.

Wet granulation can be difficult to control precisely from batch-to-batch and it may be difficult to obtain, through wet granulation, a desirable average particle size (generally less than about 500 micron) and a suitable particle size distribution.

Adjustment in particle size of a granulate can be obtained by a number of techniques which include, but are not limited to, those known collectively as "milling." Milling can reduce the average particle size. However, this increases surface area and thus the techniques (granulation and milling) must often be balanced. Moreover, milling tends to increase or widen the particle size distribution and in particular it can increase the number of small particles or "fines" in the resulting milled granulate. These "fines" (particles whose particle size and/or overall shape are not sufficiently large so as to prevent them from passing through a 105 micron screen (140 mesh)) can have deleterious effects in terms of, for example, applying a taste masking coating. Fines will behave in most coating apparatuses very differently than larger particles and accommodating both can be challenging. Moreover, fines can negatively affect flow, and compressibility and taste.

Attempting to obtain a relatively small average particle size and a narrow particle size distribution while milling generally requires the use of a small screen (a screen having relatively small openings) and such screens, even when vacuum is applied, can clog when the granulate is wet. The use of larger screens and, for example, vacuum, can improve through-put. However, it can be difficult to obtain lower average particle sizes, with desirable particle size distributions.

Thus the conundrum — how to obtain a relatively small average particle size with a narrow particle size distribution and a relatively reduced content of fines when neither wet nor dry milling appears to work. The present invention addresses at least some of the disadvantages described.

### SUMMARY OF THE INVENTION

In one aspect, the present invention includes a method of producing a wet granulate having a desirable particle size distribution comprising the steps of: drying a wet granulate ("WG") having an initial moisture content and often comprising at least one active pharmaceutical ingredient ("API"), to a predetermined first relative moisture content to form a partially dried wet granulate ("PDWG"); milling the partially dried wet granulate to obtain a milled partially dried wet granulate (also referred to herein as a "milled wet granulate," "milled granulate" or "MWG") having predetermined average particle size and/or particle size distribution; and drying the milled partially dried wet granulate to produce a final dried wet granulate ("FDWG").

In one embodiment, the predetermined first relative moisture content is at least about 30% less than the initial moisture content of the wet granulate.

In another embodiment, the final dried wet granulate has an average particle size of between about 150 and about 600 microns and no more than about 40% of the particles by weight have a particle size of less than 105 microns by weight obtained by sieving.

In still another embodiment, the predetermined first relative moisture content is about 30% less than the initial moisture content and the average particle size ranges from between about 200 to about 600 microns.

In another embodiment, the wet granulate further comprises at least one excipient which may be, without limitation, a binder, disintegrant, filler, effervescent couple, release controlling material or taste masking materials.

In yet another embodiment, the method further comprises coating the final dried wet granulate.

In one embodiment, the coating is a taste masking coating.

The final dried wet granulate produced according to any of the combined process steps, whether coated or not, are also contemplated.

In particular, one aspect of the invention is a dried wet granulate comprising: at least one pharmaceutically active ingredient ("APT") granulate and at least one excipient, the dried wet granulate having an average particle size of between about 150 and about 600 microns and no more than about 40% of the particles by weight have a particle size of less than 105 microns. Another aspect of the invention is a dried wet granulate comprising: at least one pharmaceutically active ingredient granulate and at least one excipient, the dried wet granulate having an average particle size of between about 150 and about 600 microns and no more than about 35% of the particles by weight have a particle size of less than 105 microns. In yet another aspect, the invention is a dried wet granulate comprising: at least one pharmaceutically active ingredient granulate and at least one excipient, the dried wet granulate having an average particle size of between about 200 and about 600 microns and no more than about 30% of the particles by weight have a particle size of less than 105 microns.

The dried wet granulate may further comprise at least one coating, which may be a taste masking coating, moisture barrier coating or a controlled release coating.

In one embodiment, the mill used in accordance with the present invention is a "screen mill." In another embodiment, the processes of the invention include the step of spherinizing the partially dried wet granulate prior to obtaining the final dried wet granulate.

In still another embodiment, granulates are produced that do not include an API. These granulates may be used in production of dosage forms and both the methods and the resulting non-API-containing granulates are contemplated.

### DETAILED DESCRIPTION

While the specification concludes with the claims particularly pointing and distinctly claiming the invention, it is believed that the present invention will be better understood from the following description. All percentages and ratios used herein are by weight of the total composition and all measurements made are at 25°C and normal pressure unless otherwise designated. All temperatures are in Degrees Celsius unless specified otherwise. The present invention can comprise (open ended) or consist essentially of the components of the present invention as well as other ingredients or elements described herein. As used herein, "comprising" means the elements recited, or their equivalent in structure or function, plus any other element or elements which are not recited. The terms "having" and "including" are also to be construed as open ended unless the context suggests otherwise. As used herein, "consisting essentially of" means that the invention may include ingredients in addition to those recited in the claim, but only if the additional ingredients do not materially alter the basic and novel characteristics of the claimed invention. Preferably, such additives will not be present at all or only in trace amounts. However, it may be possible to include up to about 10% by weight of materials that could materially alter the basic and novel characteristics of the invention as long as the utility of the compounds (as opposed to the degree of utility) is maintained. All ranges recited herein include the endpoints, including those that recite a range "between" two values. Terms such as "about," "generally," "substantially," and the like are to be construed as modifying a term or value such that it is not an absolute, but does not read on the prior art. Such terms will be defined by the circumstances and the terms that they modify as those terms are understood by those of skill in the art. This includes, at very least, the degree of expected experimental error, technique error and instrument error for a given technique used to measure a value.

The process of the invention begins by acting upon a wet granulate. A wet granulate in accordance with the present invention means a material that has been granulated using a binder and/or granulation liquid which can be water, a solvent, or a mixture of both. And while the process will often be described herein in terms of moisture or water (such as in references to "moisture content") it will be appreciated that such references are interchangeable with solvent and liquid as the context permits.

There are many well known granulating devices and granulating techniques and any or all thereof may be employed. Indeed, the term wet granulate can also encompass a dry granulated material that is subsequently wetted. Wet granulation can involve a number of process parameters including impeller speed, chopper speed, the length of time that wet granulate is allowed to process (measured by torque, load, or time) the type, source, and amount of granulating liquid such as water, alcohol or other solvent or co-solvent mixtures, the nature and amount of API to be used as well as its solubility with the granulating liquid as well as any other liquid or solid ingredients which may form part of the wet granulate and their relative percentages. Such other materials include, without limitation, other APIs, fillers, binders and disintegrants as well as materials such as certain types of microcrystalline cellulose which can act as both binder and disintegrant. The nature and type of the finished dosage form may also play a role in the selection of ingredients and granulation parameters. Granulating liquids that may be used include nonaqueous granulating liquids defined as an organic solvent which contains 25% or less, by volume, water or aqueous granulating liquids defined as a granulating liquid comprising more than 25% water and less than 75% of one or more suitable organic solvents.

Granulates can also include binders, diluents, disintegrants, lubricants, fillers, and carriers. The excipients may be hygroscopic or non-hygroscopic. These same materials may often also be used as excipients in producing dosage forms when blended with a granulate as described herein. Binders include but are not limited to: starch, (including corn starch and pregelatinized starch), gelatin, sugars (including sucrose, glucose, dextrose and lactose), polyethylene glycol, waxes, and natural and synthetic gums *(e.g.,* acacia, sodium alginate, polyvinylpyrrolidone, cellulosic polymers (including hydroxypropyl cellulose, hydroxypropyl methylcellulose, methyl cellulose, hydroxyethyl cellulose, and the like).

Diluents or Fillers include, but are not limited to spray-dried monohydrate or anhydrous lactose, sucrose, dextrose, mannitol, sugar alcohols, sorbitol, starch, cellulose (e.g., microcrystalline cellulose,) dihydrated or anhydrous dibasic calcium phosphate, tricalcium phosphate, maltodextrins, calcium carbonate, calcium sulfate and others.

Disintegrants include but are not limited to starches, clays, celluloses, algins, gums or cross linked polymers. Cross-linked polyvinyl pyrillidone (PVP-XL) , sodium starch glycolate and croscarmellose sodium, and effervescent agents. Effervescent agents include but are not limited to: the acid sources or acid may be any which are safe for human consumption and may generally include food acids, acid anhydrides and acid salts. Food acids include citric acid, tartaric acid, malic acid, fumaric acid, adipic acid, and succinic acids etc. Acid anhydrides and acid of the above described acids may also be used. Acid salts may include sodium, dihydrogen phosphate, disodium dihydrogen pyrophosphate, acid citrate salts and sodium acid sulfite. Carbonate sources include dry solid carbonate and bicarbonate salts such as sodium bicarbonate, sodium carbonate, potassium bicarbonate and potassium carbonate, magnesium carbonate and sodium sesquicarbonate, sodium glycine carbonate, L-lysine carbonate, arginine carbonate and amorphous calcium carbonate.

If desired the granulation may also contain minor amounts of nontoxic substances such as wetting or emulsifying agents, pH buffering agents and the like, for example, sodium acetate, sorbitan monolaurate, triethanolamine, sodium acetate, triethanolamine oleate, sodium lauryl sulfate, dioctyl sodium sulfosuccinate, polyoxyethylene sorbitan fatty acid esters, colorants, lubricants.

For present purposes, however, perhaps the most immediately important parameter of concern for the wet granulate is its initial moisture content. "Initial moisture content" ("Initial solvent content" in the case of a nonaqueous solvent and "initial liquid content" when mixed aqueous solvents are used, all of which are used synonymously herein) refers to the moisture content of the granulate immediately following granulation. This number can be determined, such as just before milling, or can be calculated based on, for example, the relative proportion of wet and dry materials. For the purpose of the present invention, however, initial moisture content will be predicated on a theoretical calculation of the amount of granulating liquid added to create the wet granulate relative to the proportion of dry ingredients.

The initial moisture content may vary significantly depending on the type of granulate, the degree of liquid materials added, and the like. Indeed, the resulting wet granulate may even be a paste without discrete particles/granules. It will be appreciated that the reduction in moisture content in accordance with the present invention is predicated on the initial moisture content not on an absolute moisture content. Thus a reduction is always measured as a percentage of the amount of initial moisture rather than the amount of actual moisture in the granulate per *se*. However, it will be appreciated, that if the moisture content of the wet granulate exiting the granulation step is too low it will, in essence, act as a dry granulate with the attendant problems previously discussed.

In accordance with one aspect of the present invention, the wet granulate is partially dried. Specifically, the wet granulate is dried to a predetermined first relative moisture content so as to produce a partially dried wet granulate or "PDWG."

The "predetermined first relative moisture content" is the moisture content of the wet granulate after at least one first drying step and it is a level which is precalculated to a desired target moisture content or range. As noted previously, this moisture content is measured as a reduction in the total initial moisture not as a function of the overall moisture content of the granulate. This can be calculated based on weight reduction, loss on drying, Karl Fischer or other known techniques. A Mettler Toledo HR-73 Moisture Analyzer was used for moisture content determinations using a default program provided by the manufacturer. However, other devices and techniques may be used.

The predetermined first relative moisture content in accordance with the present invention provides a material which is still wet, e.g., it has not been dried to completion. Moreover, it can provide a material which is desirable in terms of workability. Specifically, when the partially dried wet granulate is later milled, it is sufficiently dry to go through a relatively small screen necessary to obtain the desired average particle size and particle size distribution without clogging like a dry material and yet will not generate excessive fines, an undesirable particle size distribution or clog the screen preventing milling as an essentially undried wet granulate can.

In one embodiment, the predetermined first relative moisture content is at least about 30% less than the initial moisture content of the wet granulate. That is to say, the moisture content of the PDWG is at least about 30% less than the initial moisture content of the wet granulate.

In one embodiment, the predetermined first relative moisture content is between about 30% and about 85% less than the initial moisture content of the wet granulate. In another embodiment, the PDWG has a predetermined first relative moisture content which is between about 40% and about 80% less than the initial moisture content of the wet granulate. In yet another embodiment, the PDWG has a predetermined first relative moisture content which is between about 50% and about 80% less than the initial moisture content of the wet granulate. In still another, the PDWG has a predetermined first relative moisture content which is between about 60% and about 75% compared to the initial moisture content of the wet granulate.

The partially dried wet granulate having its predetermined first relative moisture content can be used immediately or can be stored prior to subsequent processing steps. Generally, it will be stored in a manner that retards significant further loss of moisture.

This first drying step, which results in a partially dried wet granulate, can be undertaken using any drying technology. These include, without limitation, forced air, drying the material in ambient air, drying the material in ovens on trays or racks, convection ovens, forced air ovens, tumble drying, infra-red or microwave drier, drying in a jacketed granulator, drying using vacuum or in a fluidized bed. Note, that "predetermined" in this regard can mean a specific established target. It can also, particularly in research settings, be an unspecified amount that is short of substantially complete drying as discussed herein. It is also possible to use multiple drying steps and/or apparatus so as to reach the predetermined first relative moisture content. For one non-limiting example, one can employ a particular drying apparatus to obtain a reduced moisture content which is an approximation of the target and allow the material in question to dry in the open air or through some other device or technique to obtain the desired predetermined first relative moisture content.

After the first partial drying step (which, as noted above, can be multiple steps), at least a portion of the PDWG with a predetermined first relative moisture content is milled. By at least a portion, it is understood that not all of the PDWG must be milled -- the balance put aside and often eventually recombined with the milled PDWG. For example, in one embodiment where the wet granulate includes fexofenadine, the PDWG was first screened through a 30 mesh (590 micron) and 40 mesh (420 micron) screen. Those particles that go through the 40 mesh screen are removed and put aside. Those particles that are retained on the 40 mesh screen and, in this instance, those which are retained on the 30 mesh screen, are milled as disclosed herein. These two fractions, and indeed any others, can be milled and dried together or separately. After milling, they are recombined with the granulate that went through the 40 mesh screen and the second drying step initiated.

These two portions could also be dried separately and recombined or, used completely separately. In the latter instance only, it is the average particle size of the milled materials and that fraction's particle size distribution that are considered for purposes of the invention. When the two fractions are recombined, one considers the average particle size and distribution of the recombined milled and unmilled materials.

In multiple batches with this active, the average particle size prior to coating ranged from about 196 to about 279 microns as measured by sieving based on a determination by weight using the following screens: 30,40, 50, 60, 80, 100, 120 mesh and the pan. In these instances, no more than about 35% by weight would go through a 105 micron screen.

The processes in accordance with the present invention have also been used in connection with the production of tablets containing desloratadine containing granules. The granulate produced with this active was not divided as previously described. Instead, substantially the entire granulate was milled and subsequently dried together. Average or mean particle size measurements based on the final dried granulate range from between about 220 and about 250 microns as determined by using the following screens: 35, 40, 45, 50, 60, 70, 80, 100, 120 mesh screens and a pan. It is not essential that this number or these exact screens be used in connection with average particle size and/or particle size distribution determinations for any particular active. The foregoing are just suggestions. Any number and type of screens may be used which provide a sufficiently accurate mean or average number. Other automated techniques may also be used.

This is a particularly useful technique to use when, for example, relatively softer granulates are produced. In the above example, if the wet granulate retained on the 30 and 40 mesh screens were dried completely, and milled, the number of fines would grow significantly. Even if this material was added to the particles that went through both the 30 and 40 mesh screens, the resulting average particle size would be affected and the number of fines could exceed 40, or even 50% by weight. And milling the smaller particles, even if undertaken on only partially dried granulates, could also adversely effect particle size and distribution. It is possible to reduce the number of fines and alter the average particle size by removing a sieve cut of material. But that can seriously effect yield. In one embodiment, the use of the present invention limits the need to discard a portion of the granulate and even more preferably, no more than about 10% is intentionally discarded (although there may be additional losses due to normal processing issues).

After the first partial drying step (which, as noted above, can be multiple steps), at least some portion of, and preferably substantially all of the PDWG with a predetermined first relative moisture content is milled. As previously noted, for that portion of the PDWG that is milled, milling can reduce particle size and assist in obtaining the desired particle size distribution and average particle size. However, the process has been restricted generally to dry materials especially where it is desirable to obtain relatively small average particle sizes.

Mills that may be used include, without limitation, impact mills such as Hammer Air Swept mills (Alpine, Bepex, Sturtevant); Hammer Conventional mills (Alpine, Fitzpatrick, Fluid Air, Mikro, Rietz, Stokes-Merrill); Pin/Disc mills (Alpine, Kemutec, Sturtevant); and Cage mills (Stedman) ; cutting mills (Alpine, Fitzpatrick, Urschel); and screening mills such as Rotating Impeller mills (Bepex, Fitzpatrick, Fluid Air, Jetpharma, Kemutec, Quadro), Stokes-Merrill and Zanchetta mills; Rotating Screen mills (Glatt); and Oscillating Bar mills (Bepex, Frewitt, Jackson-Crockatt, Stokes-Merrill, Vector). Any other type of milling mill and technique may also be used to obtain the desired particle size, particle size distribution and fines content. However, for many reasons, including those described herein, a fitzmill or other device employing a screen may be preferred.

When milling the partially dried wet granulate in accordance with the present invention, there are several variables which must be considered. These include the volume and throughput of the material, whether a vacuum will be applied, the configuration of the mill, the size of the screen used (where appropriate), the number of bars or knives used and their disposition as well as the speed of the mill and clearance between the impeller and the screen. Many of the parameters used in milling will vary depending upon the type of mill used and the material being milled. However, in general, and as an illustration of the types of conditions used, an impeller-based mill such as a fitz mill will use an impeller speed of at least about 1,000 RPM, more preferably 1,000-5,000 RPM, most preferably 2,500-3,000 RPM. Screens used can range from about 20 mesh (840 microns) to about 140 mesh (105 microns), as long as the particle size and particle size distribution of the present invention are realized. Also of particular importance are the average particle size and particle size distribution that are desired, which can be measured at many places along the process, but is generally measured after the completion of the final drying step. Any combination of milling device, speed and configuration that can provide granulates of the present invention with desirable particle size distribution and average particle size are contemplated. Desirable in terms of average particle size means the average particle size will generally range from between about 150-600 microns, more preferably 175-600 microns and most preferably between about 200 to about 600 microns, knowing full well that most granulation techniques would be unable to provide average particle size within the majority of this range without the creation of too many fines and a disadvantageous particle size distribution. Too many fines as used herein is one measure of particle size distribution and generally refers to that number of fine particles (those that pass through a 105 micron screen) which exceeds approximately 40%, more preferably 35% and even more preferably 30% by weight as measured by sieving.

While any milling device which can provide the advantages of particle size and particle size distribution in accordance with the present invention may be used, the use of fitzmill or other screen containing device can be particularly advantageous. Without wishing to be bound by any particular theory of operation, it is believed that the screen used in a fitzmill can act as a extruder. The still moistened materials in the mill are far more malleable than a dried or substantially dried material and it is believed that the act of forcing the material through the screen or other similar structure can begin the process known as spherinization. Spherinization is a term used in the pharmaceutical industry for processing granulated or particulate material into a more spherical shape thus facilitating its useful properties; particularly its properties in handling and in formulating dosage forms. A mill in this context (collectively referred to herein as a "screen mill") which employs a screen or other device that can function to extrude the PDWG or can be used in combination with an immediately downstream screen, is a preferred embodiment and it is surprising that the use of such screen mills facilitates spherinization.

It is also surprising that by the use of the present invention, one is able to obtain effective milling even though the granulate being milled remains wet. It has been determined, however, that unless the initial moisture content is reduced to a predetermined first moisture content which is at least about 30% less than the initial moisture content, milling can be unsuccessful in that it may be unprocessable through the screen and/or may create too many fines and provide an undesirable average particle size and/or particle size distribution.

Milling can be accomplished in open air, in an inert environment, or under vacuum.

Once milling has been accomplished, the now milled partially dried wet granulate (also referred to as the milled wet granulate, milled granulate, or "MWG") is further dried. Actually, the drying process can begin during milling. For example, when a vacuum is applied, this process can further desiccate the material thus effecting a further drying. However, the step of drying the milled partially dried wet granulate so as to produce a final dried granulate ("FDWG") is intended to withdraw sufficient moisture from the milled wet granulate so as to produce a final dried wet granulate having the desired target final moisture content. This does not mean that the FDWG must be completely dry or that it cannot and/or will not gain water subsequently. Materials used in the pharmaceutical industry may be hygroscopic and may attract water once a drying operation is complete, depending on storage and other considerations. Thus the final dried wet granulate need not be completely dry but only "substantially" dry. Substantially dry in accordance with the present invention means that the moisture content of the final dried wet granulate has been reduced to a second predetermined and desirable level generally about 15% based on the initial moisture content, or less. This final moisture level is preferably stable in normal packaging under normal conditions.

This second drying step can be accomplished in any manner as previously described for the first drying step. Material may be allowed to air dry, it may be desiccated using normal desiccants, dried in an oven, vacuum dried or dried in a fluidizer bed. Drying may be accomplished in one or more devices and/or one or more steps.

Both the predetermined first relative moisture content and the final moisture content may be determined as previously described.

In a particular preferred embodiment in accordance with the present invention milling and/or drying devices are used which facilitate spherinization. This concept has already been briefly discussed in the context of milling where it was described that the use of a "screening mill" would be desirable. It has been found, however, that spherinization can be further facilitated during the second or final drying step. The partially dried wet granulate that has now been milled, and in particular through a screen mill, can be directly transferred into, for example, a fluidize bed drying device. The still partially wet material enters the fluidize bed and is circulated throughout it, both by the action of additional loading and by the use of the air and vacuum being used within the bed. Accordingly, it may be necessary or desirable to maintain the material in the fluidized bed for a period of time that is greater than optimal for drying but which accomplishes both the appropriate level of drying and spherinization. To this end, it may be desirable to remove the spherinized particles from the fluidized bed before they are completely dried allowing drying to continue through other techniques. Spherinization can be determined at least subjectively by microscopic analysis comparing material immediately after milling with material that has been further processed as described herein.

Because a fluidized bed can be used for both the initial drying and final drying steps, it may be desirable to use the same fluidized bed for both drying steps, allowing for more efficient processing.

It has also been discovered that spherinization can be further influenced by the manner in which the partially dried milled granulate is transferred from the mill to the final drying apparatus. In one particular preferred embodiment, this transfer occurs dynamically and preferably continuously during milling through the use of a vacuum tube. It has been found that within the vacuum hose itself, the still moistened milled wet granulate becomes further spherinized. Without wishing to be bound by any particular theory of operation it is believed that as the milled wet granulate is drawn through the vacuum tubing, it is forced into contact with the inner wall of the tubing which is a curved surface and that rolling along that curved surface further facilitates spherinization.

Indeed, in one embodiment, the transfer tubing may serve as the spherinizing device and final drying may not be involved in spherinizing. Thus, after milling, the partially dried wet granulate can be transferred into a hose, preferably a vacuum hose, which is sufficiently sized in length and inner diameter so as to spherinize the partially dried wet granulate. Once the spherinized partially dried wet granulate exits the transfer hose, it could empty into trays for a static final drying step in an oven. This latter drying step will not improve spherinization.

Preferably, the vacuum tubing used for this type of transfer tends to facilitate spherinization and has an inside diameter ranging from between about 1 to about 6 inches and has a length of between about 4 to about 100 feet. It will be appreciated that the degree of vacuum applied, the length of the tube and its inside diameter are all interdependent and can depend on other factors including the size of the batch, the type of equipment being used, and the like. Less vacuum, smaller inside diameter tubes and shorting tubing may be necessary for pilot scale batch production compared to production scale equipment. Moreover, while the length of the tube just described has been indicated as being upwards of 100 feet long, it will be appreciated that the upper limit is really unimportant so long as it is sufficiently long to allow interaction to the appropriate equipment, to assist in spherinization and to allow for efficient processing. The degree of vacuum used can vary significantly depending on a number of factors, including the type of equipment, batch size, inside diameter of the tubing, and the like. In one particular embodiment, the vacuum generated by use of 105 horsepower fan load generated by a magnacoater fluidized bed apparatus manufactured by fluid air wherein a batch was drawn from a 6 inch interdiameter tubing of about 15 feet in length was more than adequate for efficient transfer and provided additional benefits from terms of spherinization. A vacuum equivalent to that which would be generated from the same device at approximately 80 horsepower fan load would be also useful. Of course, a vacuum can be generated by any number of means and this is offered only by way of illustration.

It should also be noted that, in addition to or instead of the use of transfer tubing, traditional spherinization apparatus can be used in between the milling and the final drying step to accomplish spherinization. Thus the milled wet granulate could be emptied into a spherinization pan or other device, spherinized, and then placed into a drying apparatus for subsequent drying.

After the subsequent drying step, the final dried wet granulate should have an average particle size as measured as previously discussed of between about 150 to about 600 microns. More preferably, the average particle size and the resulting FDWG will have an average particle size of between about 175 and about 600 microns. More preferably, the final dried wet granulate will have an average particle size ranging between about 200 to about 600 microns. This is measured by weight based on sieving as previously described.

The particle size distribution of the resulting final dried wet granulate can generally be characterized as resulting in a material having about 40% of the particles, or less, by weight, which will pass through a 105 micron screen. Even more preferably, the material should have no more than about 30% of the particles sufficiently small to pass through a 105 micron screen. However, this is not the only way to characterize the particle size distribution of the resulting materials. More preferably, no more than about 35% of the particles by weight have the particle size sufficiently small such that they pass through 105 micron screen. Another way to describe an advantageous particle size distribution in accordance with the present invention is that not less than about 60% of the particles (w/w) fall within 50% of the mean or average particle size as.determined. Most preferably, no more than 40% of the particles will be sufficiently small to pass through 105 micron screen and not less than about 60% of the particles w/w will be within 50% of the average particle size.

Another processing step useful in accordance with the present invention is coating. Coating can be accomplished in the same fluidized bed as drying and/or spherinization, can be accomplished using a second fluidized bed in communication with the first fluidized bed (or discreetly) or can be accomplished using any other coating device and/or technology available. Coating can be undertaken for any number of reasons, one of which is taste masking. However, coating may also be undertaken for, for example, providing a controlled release. Controlled release in accordance with the present invention can mean a rapid release in the stomach (a release profile similar to that which would result from taking the same amount of pharmaceutical active ingredients as an uncoated powder or particulate) or quickly upon entering the appropriate portion of, for example, the intestines (as in the case ot enteric coating which releases quickly once the proper pH environment is reached) . Controlled release can also mean a timed-release coating which extends the release over a period of time. Indeed, it may be possible to provide an enteric coating which not only delays the release of the active pharmaceutical ingredient until the dosage form reaches the desired portion of the intestines, but also may provide for an extended period of release while traversing the intestines.

Any suitable coating technique may be used and any suitable coating may be used. For taste masking, coating may be accomplished as described in U.S. Patent Nos. 5,178,878 and 6,740,341. For controlled release coatings, including enteric coatings, any coating material may be used such as those described in U.S. Patent Nos. 6,787,155; 6,635,680; 6,077,533; and 6,572,885.

Methods of encapsulation or microencapsulation (methods of coating) which may be used include, without limitation, those described in Lieberman et al., Pharmaceutical Dosage Form: Tablets Volume 1, Second Edition, New York, 1989, at pages 372-376. That disclosure is hereby incorporated by reference herein. One method taught in Lieberman is the technique of phase separation or coacervation which involves processing three mutually immiscible phases, one containing the pharmaceutical ingredient, another containing the protective coating material and a third containing a liquid vehicle used only in the manufacturing phase. The three phases are mixed and the protective material phase deposits by absorption on the pharmaceutical ingredient phase. After this step, the protective material phase is converted to a substantially solid form by cross-linking or by removal of solvent from this phase. Alternatively, the mixture of the pharmaceutical ingredient and polymeric solution may be mixed with an immiscible liquid phase and the solvent may be removed through this phase. The mixing step may include emulsification of the phase bearing the pharmaceutical ingredient and the protective material in the immiscible liquid phase. Barrier coating materials include without limitation: modified celluloses such as ethyl cellulose, methyl cellulose, hydroxypropyl cellulose, hydroxypropyl methylcellulose, polyalkylene glycols, polyalkylene oxides, sugars and sugar alcohols, waxes, shellacs, acrylics, etc. and mixtures thereof. Other coating materials include without limitation: Anti-tack agents, fillers, plasticizers, pore forming agents, glossing agents, colorant, polymethacrylates (e.g., Rohm Eudragit products), ethylcelluloses, celluloses, hydroxypropyl cellulose, hydroxypropyl methylcellulose, sugars, sugar alcohols, waxes, shellacs, acrylics and mixtures thereof.

Spray coating in a suitable apparatus may also be used.

The coating material may incorporate polymers such as those conventionally utilized in coating. A wide variety of polymers are known for this purpose. Any such known polymeric material, utilized heretofore in production of microcapsules and coated particles may be employed. Among these are cellulosic materials such as naturally occurring cellulose and synthetic cellulose derivatives; acrylic polymers and vinyl polymers. Other suitable polymers include proteinaceous materials such as gelatin, polypeptides and natural and synthetic shellacs and waxes. Particularly preferred protective material polymers include ethylcellulose, methylcellulose, carboxymethylcellulose and the acrylic resin materials sold under the registered trademark EUDRAGIT by Rhone Pharma GmbH of Weiterstadt, Germany.

Many of the coating material polymers discussed above have substantial resistance to dissolution in water. Such water-insoluble materials can be used to make controlled release coated granulates. Preferably, however, where the coating material incorporates water-insoluble materials of this nature, it also includes other ingredients to promote more rapid release of the API. Such release promoters include soluble polymers and, in particular, polyfractional alcohols such as mannitol, as well as magnesium oxide. For example, the acrylic material of the type known as EUDRAGIT^{®} RL30-D, when used with conventional coingredients such as methylcellulose and magnesium stearate tends to provide a slow release, typically about 50 percent or less after 30 minutes. However, a coating material incorporating the same polymeric material in conjunction with about 2 to about 4, and preferably about 2.7 parts mannitol per part EUDRAGIT material on a solids basis, and also incorporating about 0.05 to about 0.2, and preferably about 0.09 parts magnesium oxide per part EUDRAGIT solids provides a protective material with substantially immediate release properties. Blends of acrylic polymers such as EUDRAGIT with polyfunctional alcohol such as mannitol, and, desirably, with oxides of alkaline earth metals such as magnesium oxide provide prompt release of the API. Such blends do not include the plasticizers commonly used with acrylic protective materials. Granulates coated using such blends normally are susceptible to release upon chewing, but nonetheless provide excellent taste-masking properties in tablets according to this invention.

The type of coating and the amount of coating material used will vary depending upon, among other things, the type of active material, the goal of coating (taste masking, time release, etc.), the volume of the resulting dosage form, the properties that the coating may impart in terms of formulating a dosage form, and the type of coating material used. However, generally the coating will be applied such that, overall, the weight gain of the final dried wet granulate increases by from about 0.10 to about 300% by weight, or preferably from between about 1 to about 200% by weight per layer. Eudragit E-100 is particularly preferred in an amount of 1-50%.

The final dried wet granulate, whether subsequently coated or not, can then be formulated into dosage forms just as any other wet or dry granulate. The granulate preferably can be used in a direct compression process whereby it is mixed with other tableting excipients such as fillers, binders, disintegrants, lubricants, flavors, colors, flow enhancers, and the like in, for example, a V blender and blended for a sufficient period of time to provide substantial homogeneity. The material can then be compressed into tablets. Alternatively, the granulate, either alone or with other agents or excipients, can be filled into capsules or used in any other appropriate dosage form.

APIs in accordance with the present invention include pharmaceuticals, vitamins, minerals, dietary supplements and the like. Pharmaceuticals include, without limitation those disclosed in Mantelle, U.S. Patent No. 5,234,957, and in particular, in columns 18-21 may be used and they are hereby incorporated by reference. Classes of APIs include:
Abortifacient/Interceptive
Ace-Inhibitor
α - Adrenergic Agonist
β - Adrenergic Agonist
α - Adrenergic Blocker
β - Adreneregic Blocker
Adrenocortical Steroid
Adrenocortical Suppressant
Adrenocorticotropic Hormone
Alcohol Deterrent
Aldose Reductase inhibitor
Aldosterone Antagonist
5-Alpha Reductase Inhibitor
Ampa Receptor Antagonist
Anabolic
Analeptic
Analgesic (Dental)
Analgesic (Narcotic)
Analgesic (Non-Narcotic)
Androgen
Anesthetic (Inhalation)
Anesthetic (Intravenous)
Anesthetic (Local)
Angiotensin Converting Enzyme Inhibitor
Angiotensin II Receptor Antagonist
Anorexic
Antacid
Anthelmintic (Cestodes)
Anthelmintic (Nematodes)
Anthelmintic (Schistosoma)
Anthelmintic (Trematodes)
Antiacne
Antiallergic
Antiallergic (Hyposensitization Therapy)
Antiallergic (Steroidal Nasal)
Antialopecia Agent
Antiamebic
Antiandrogen
Antianginal
Antiarrhythmic
Antiarteriosclerotic
Antiarthritic/Antirheumatic
Antiasthmatic (Nonbronchodilator)
Antiasthmatic (Steroidal, Inhalant)
Antibacterial (Antibiotics)
Antibacterial (Synthetic)
Antibacterial (Leprostatic)
Antibacterial (Rickettsia)
Antibacterial (Tuberculostatic)
Antibacterial adjuncts
Antibiotic
Anticancer
Anticholelithogenic
Anticholesteremic
Anticholinergic
Anticoagulant
Anticonvulsant
Antidepressant
Antidiabetic
Antidiarrheal
Antidiuretic
Antidote (Acetaminophen Poisoning)
Antidote (Curare)
Antidote (Cyanide)
Antidote (Folic Acid Antagonists)
Antidote (Heavy Metal Poisoning)
Antidote (Methanol and Ethylene Glycol Poisoning)
Antidote (Organophosphate Poisoning)
Antidyskinetic
Antieczematic
Antiemetic
Antiepileptic
Antiestrogen
Antifibrotic
Antiflatulent
Antifungal (Antibiotics)
Antifungal (Synthetic)
Antiglaucoma
Antigonadotropin
Antigout
Antihemophilic Factor
Anthemorrhagic
Antihistaminic
Antihypercholesterolemic
Antihyperlipidemic
Antihyperlipoproteinemic
Antihyperparathyroid
Antihyperphosphatemic
Antihypertensive
Antihyperthyroid
Antihypotensive
Antihypothyroid
Anti-Infective
Anti-Inflammatory (Gastrointestinal)
Anti-Inflammatory (Nonsteroidal)
Anti-Inflammatory (Steroidal)
Antileprotic
Antileukemic
Antilipemic
Antilipidemic
Antimalarial
Antimanic
Antimethemoglobinemic
Antimigraine
Antimuscarinic
Antmycotic
Antinauseant
Antineoplastic
Antineoplastic (Hormonal)
Antineoplastic (Photosensitizer)
Antineoplastic (Radiation Source)
Antineoplastic Adjunct
Antineurtropenic
Antiobesity Agent
Antiobsessional
Antiosteroporotic
Antipagetic
Antiparkinsonian
Antiperistaltic
Antipheochromocytoma
Antipneumocystic
Antiprogrestin
Antiprostatic Hypertrophy
Antiprozotozoal (Ameba)
Antiprotozoal (Cryptosporidium)
Antiprotozoal (Giardia)
Antiprotozoal (Leishmania)
Antiprotozoal (Malaria)
Antiprotozoal (Pneumocystis)
Antiprotozoal (Toxo-Plasma)
Antiprotozoal (Trichomonas)
Antiprotozoal (Trypanosoma
Antipruritic
Antisoriatic
Antipsychotic
Antipyretic
Antirheumatic
Antirickettsial
Antiseborrheic
Antisepsis
Antiseptic/Disinfectant
Antispasmodic
Antisyphilitic
Antithrombocythemic
Antithrombotic
Antitubercular
Antitumor
Antitussive
Antiulcerative
Antiurolithic
Antivenin
Antivertigo
Antiviral
Anxiolytic
Arosmatase Inhibitors
Astringent
Atriopeptidase Inhibitor
Benzodiazepine Antagonist
Beta-Blocker
Blood Substitute
Bone Resorption Inhibitor
Bradycaric Agent
Bradykinin Antagoinist
Bronchodilator
Calcium Channel Blocker
Calcium Regulator
Calcium Supplement
Cancer Chemotherapy
Capillary Protectant
Carbonic Anhydrase Inhibitor
Cardiac Depressant (Antiarrhythmic)
Cardioprotective
Cardiotonic
Cathartic
Cation-Exchange Resin
CCK AntagonistCentral Stimulant
Cerebral Vasodilator
Chelating Agent
Cholecystokinin Antagonist
Cholelitholytic Agent
Choleretic
Cholinergic
Cholinesterase Inhibitor
Cholinesterase Reactivator
CNS Stimulant
Cognition Activator
COMT Inhbitor
Contraceptive (Implantable)
Contraceptive (Injectable)
Contraceptive (Oral)
Control of Intraocular Pressure
Converting Enzyme Inhibitor
Coronary Vasodilator
Cyclooxygenase-2 Inhibitor
Cytoprotectant (Gastric)
Debriding Agent
Decongestant
Dental Plaque Inhibitor
Depigmentor
Dermatitis Herpetiformis Suppressant
Diagnostic Aid
Diagnostic Aid (MRI Contrast Agent)
Diagnostic Aid (Radioactive Imaging Agent)
Diagnostic Aid (Radiopaque Medium)
Diagnostic Aid (Ultrasound Contrast Agent)
Digestive Aid
Disinfectant
Diuretic
Dopamine Receptor Agonist
Dopamine Receptor Antagonist
Ectoparasiticide
Electrolyte Replenisher
Emetic
Endothelin Receptor Antagonist
Ekephalinease Inhibitor
Enzyme
Enzyme Cofactor
Enzyme Inducer (Hepatic)
Enzyme Replacement Theraphy
Estrogen
Estrogen Antagonist
Expectorant
Fibrignogen Receptor Antagonist
Gastric and Pancreatic Secretion Stimulant
Gastic proton Pump Inhibitor
Gastric Secretion Inhibitor
Gastroprokinetic
Glucocorticoid
α-Glocosidase Inhibitor
Gonad-Stimulating Principle
Growth Hormone Antagonist/Inhibitor
Growth Hormone Releasing Factor
Growth Stimulant
Hematinic
Hematopoietic
Hemolytic
Hemorheologic Agent
Hemostatic
Heparin Antagonist
Hepatoprotectant
Histamine H1-Receptor Antagonist
Histamine H2-Receptor Antagonist
HIV Fusion Inhibitor
HIV Protease Inhibitor
HMG COA Reductase Inhibitor
Hypnotic
Hypocholesteremic
Hypolipidemic
Hypotensive
Immunomodulator
Immunosuppressant
Insulin Sensitizer
Ion-Exchange Resin
Keratolytic
Lactation Stimulating Hormone
Laxative/Cathartic
Leukotrine Antagonist
LH-RG Agonist
LH-RH Antagonist
Lipotropic
5-Lipoxygenase Inhibitor
Local Anesthetic
Lupus Erythematosus Suppressant
Major Tranquilizer
Matrix Metalloproteinase Inhibitor
Mineralocorticoid
Minor Tranquilizer
Miotic
Monoamine Oxidase Inhibitor
Mucolytic
Muscle Relaxant (Skeletal)
Muscle Relaxant (Smooth)
Mydriatic
Narcotic Analgesic
Narcotic Antagonoist
Nasal Decongestant
Neuraminidase Inhibitor
Neuroleptic
Neuromuscular Blocking Agent
Neutral Endopeptidase Inhibitor
Neuroprotective
NMDA Receptor Antagonist
Nootropic
NSAID
Opioid Anagesic
Oral Contraceptive
Ovarian Hormone
Oxytocic
Parasympathomimetic
Pediculicide
Pepsin Inhibitor
Perpheral Vasodilator
peristaltic Stimulant
Phosphodiesterase Inhibitor
Pigmentation Agent
Plasma Volume Expander
Platelate Activating Factor Antagonist
Potassium Channel Activator/Opener
Potassium Challel Blocker
Pressor Agent
Progestogen
Prolactin Inhibitor
Prostaglandin/Prostaglandin Analog
Protease Inhibitor
Proton Pump Inhibitor
Pulmonary Surfactant
5α-Reductase Inhibitor
Replenishers/Supplements
Respiratory Stimulant
Retroviral Protease Inhibitor
Reverse Transcriptase Inhibitor
Scerosing Agent
Sedative/Hyponotic
Serenic
Serotonin Noradrenaline Reuptake Inhibitor (SNRI)
Serotonin Receptor Agonist
Serotonin Receptor Antagonist
Serotonin Uptake Inhibitor
Sialagogue
Skeletal Muscle Relaxant
SNRI
Somatostatin Analog
Spasmolytic
Stool Softener
Succinylcholine Synergist
Sympathomimetic
Thrombolytic
Thromboxane A2-Synthetase Inhibitor
Thyroid Hormone
Thyroid Inhibitor
Thyrotropic Hormone
Tocolytic
Topical protectant
Topoismerase I Inhibitor
Topoisomerase II Inhibitor
Tranquilizer
Ultraviolet Screen
Uricosuric
Vadodilator (Cerebral)
Vasodilator (Coronary)
Vasodilator (Periopheral)
Vasopeptidase Inhibitor
Vasopressor
Vasoprotectant
Vitamin/Vitamine Source Vulnerary Wilson's Disease Treatment Xanthine Oxidase Inhibitor.

In particular, APIs may include fexofenadine, desloratadine, tramadol, modafinil, armodafinil, clozapine, azithromycin, oxycodone, fentanyl and salts thereof.

In one embodiment, the dosage form includes a final dried wet granulate which may be spherinized and/or coated with a taste masking or controlled release coating and an effervescent agent or effervescent couple as an excipient. Any known effervescent agent or effervescent couple combination may be used. These include those described in U.S. Patent Nos. 5,178,878 and 5,503,846, the texts of which are hereby incorporated by reference to the extent they discuss various effervescent couples and constructions of same. Effervescent couples generally are water- or saliva-activated materials usually kept in an anhydrous state with little or no absorbed moisture or in a stable hydrated form. Typically these involve at least one acid source and at least one source of a reactive base, usually a carbonate or bicarbonate. Each of the components of the effervescent couple may be any which are safe for human consumption.

The acids generally include food acids, acid anhydrides and acid salts. Food acids include citric acid, tartaric acid, malic acid, fumeric acid, adipic acid, ascorbic acid and succinic acid. Acid anhydrides or salts of these acids may be used. Salts in this context may include any known salt but in particular, sodium, dihydrogen phosphate, disodium dihydrogen phosphate, acid citrate salts and sodium acid sulfate. Bases useful in accordance with the invention typically include sodium bicarbonate, potassium bicarbonate and the like. Sodium carbonate, potassium carbonate, magnesium carbonate and the like may also be used to the extent they are used as part of an effervescent couple. However, they are more preferably used as a pH adjusting substance. Preferably, stoichiometric equivalent amounts of acid, acid anhydride or acid salt and base are used. It is possible, however, that some excess of acid or base be used.

The amount of effervescent material useful in one aspect of the invention is an effective amount and is determined based on the amount necessary to provide suitable disintegration properties (time and organoleptic feel) in the mouth of a patient. However, in another aspect, effervescence may also be used as a driving force for enhancing transmission of an API, such as fentanyl across mucosal membranes via buccal, gingival, rectal, vaginal, nasal or sublingual administration or even through the stomach or intestines. In that instance, the amount of effervescent couple will be dictated by something other than the amount necessary to allow the dosage form, usually a tablet, to disintegrate. In either instance, the amount should range from between about 5 to about 85 percent, more preferably between about 15 to 60 percent, even more preferably between about 30 and 45 percent and most preferably between about 35 to about 40 percent, based on the weight of the total formulation. Of course, when used to enhance penetration, the relative proportion of acid base will depend upon the specific ingredients (for example, whether the acid monoprotic, diprotic or triprotic) relative molecular weights, etc. However, preferably, a stoichiometric amount of each is provided although, of course, excesses are acceptable.

Formulations in accordance with the present invention may also include as an excipient at least one pH adjusting substance. Without wishing to be bound by any particular theory, a pH adjusting substance may permit a drug (also referred to herein as an API) which is susceptible to changes in ionization state can be administered by ensuring the proper conditions for its dissolution as well as transmission across one or more of the membranes or tissues such as across a mucosa. pH adjusting substances in accordance with the present invention can be used to provide further permeation enhancement. The selection of the appropriate pH adjusting substance will depend on the drug to be administered and, in particular, to the pH at which it is ionized or unionized, and whether the ionized or unionized form facilitates transmission across the mucosa. pH adjusting substances in accordance with the present invention can include, without limitation, any substance capable of adjusting the localized pH to promote transport across a mucosal membrane in amounts which will result in a pH's generally ranging from between about 3 to 10 and more preferably between about 4 to about 9. The pH is the "localized pH" at the microenvironment in the mouth of a patient at the surface contact area of the mucosa and the dosage form or any portion thereof (such as when it disintegrates). For purposes of this invention, the localized pH can be determined as follows: to characterize the dynamic pH changes displayed by the tablets in question, an in vitro pH measurement was used. The method consists of using 0.5-10 mL of phosphate buffered saline in an appropriately sized test tube or similar vessel. The amount of media is dependent on the tablet size and dosage. For example, when measuring the pH profile for fentanyl tablets, a volume of 1 mL was used for tablets which weighed 100 mg. Immediately upon tablet contact with the media, the pH profile of the solution is monitored as a function of time, using a micro-combination pH electrode.

Preferably, the materials which can be used as pH adjusting substances in accordance with the present invention include carbonates such as sodium, potassium or calcium carbonate or a phosphate such as calcium or sodium phosphate. Most preferred is sodium carbonate. The amount of pH adjusting substance useful in accordance with the present invention can vary with the type of pH adjusting substance used, the amount of any excess acid or base from the effervescent couple, the nature of the remaining ingredients and, of course, the API.

Most preferably the amount of pH adjusting substance will range from between about 0.5 to about 25 percent, more preferably between about 2 to about 20 percent, even more preferably between about 5 to about 15 percent and most preferably between about 7 to about 12 percent by weight based on the weight of the total formulation. The most preferred pH adjusting substance is a carbonate, bicarbonate, or phosphate. Also preferred are those pH adjusting substances which, when provided in a suitable amount, can provide a change in the localized pH of at least about 0.5 pH units, more preferably about 1.0 pH units and even more preferably about 2.0 pH units when compared to an otherwise identical formulation without the pH adjusting substance.

Any filler or any amount of a filler may be used as an excipient as long as the resulting dosage forms achieve the results described herein. Most preferred amongst the fillers are sugar and sugar alcohols and these may include non-direct compression and direct compression fillers. Non-direct compression fillers generally, at least when formulated, have flow and/or compression characteristics which make them impractical for use in high speed tableting process without augmentation or adjustment. For example, a formulation may not flow sufficiently well and therefore, a glidant such as, for example, silicon dioxide may need to be added.

Direct compression fillers, by contrast, do not require similar allowances. They generally have compressibility and flowability characteristics which allow them to be used directly. It is noted that, depending upon the method by which formulations are made, non-direct compression fillers may be imparted with the properties of direct compression fillers. The reverse is also true. As a general matter, non-direct compression fillers tend to have a relatively smaller particle size when compared to direct compression fillers. However, certain fillers such as spray dried mannitol have relatively smaller particle sizes and yet are often directly compressible, depending upon how they are further processed. There are also relatively large nondirect compression fillers as well.

Fillers that are preferred in accordance with the present invention include mannitol, lactose, sorbitol, dextrose, sucrose, xylitol and glucose, to the extent their use can provide the results described herein. Most preferred in accordance with the present invention, spray dried mannitol is used. The amount of filler can range from 10 to about 80% and more preferably about 25 to about 80%, most preferably 35 to about 60% by weight of the formulation.

Noneffervescent disintegrants may also be used in accordance with the present invention as excipients. These may also include binders that have disintegrating properties. Disintegrants in accordance with the present invention can include microcrystalline cellulose, cross-linked polyvinyl pyrrolidone (PVP-XL), sodium starch glycolate, croscaramellose sodium, cross-linked hydroxypropyl cellulose and the like.

The amount of noneffervescent disintegrant will vary with known factors such as, the size of the dosage form, the nature and amounts of the other ingredients used, etc. However, in general the amount should range from between about 0.25 to about 20% by weight of the final formulation, more preferably between about 0.5 to about 15% w/w, even more preferably 0.5 to about 10% w/w and even more preferably between about one and about eight percent by weight. This is again based on the weight of the finished formulation.

Also generally useful as an excipient in accordance with the present invention is a tableting or ejection lubricant. The most common known lubricant is magnesium stearate and the use of magnesium stearate is preferred. Generally, the conventional wisdom behind tableting lubricants is that less is more. It is preferred in most circumstances that less than about one percent of a tableting lubricant be used. Typically, the amount should be half a percent or less. However, the amount of magnesium stearate used can be greater than 1.0%. Indeed, it is preferably greater than about 1.5% and most preferably between about 1.5% and about 3%. Most preferred is the use of about 2% magnesium stearate. Other conventional tableting lubricants such as, for example, stearic acid, calcium stearate and the like may also be used in place of some or all of the magnesium stearate.

Tablets in accordance with the present invention can be relatively soft or robust. They can, for example, be manufactured in accordance with the methods described in U.S. Patent No. 5,178,878 and will have a hardness of generally less than about 15 Newtons. The '878 patent is incorporated herein by reference not only for its description of tablet manufacture, but also coating of a an API-containing granulate. When tablets as soft and pliable/friable as these are produced, they may be advantageously packaged in a blister package such as found in U.S. Patent No. 6,155,423. They may also be robust with a hardness of greater than about 15 Newtons, manufactured in accordance with the procedures set forth in U.S. Patent No. 6,024,981, which is also hereby incorporated by reference.

The formulations in accordance with the present invention can include other conventional excipients in generally known amounts (usually up to about 20% by weight) to the extent they do not detract from the advantages described herein. These can include without limitation binders, sweeteners, coloring components, flavors, glidants, preservatives, and the like.

Tablets, a preferred dosage form in accordance with the present invention, can be made by any known tableting technique including those described above in the '878 and '981 patents which are incorporated herein by reference for their disclosures of tablet manufacture. However, preferably, the materials used, including the final dried wet granulate, are dry blended and directly compressed.

Of course, particular excipients and materials used in formulations in accordance with the present invention may be wet or dry granulated separately from the API. If wet granulated, they are preferably wet granulated as described herein. These non-API-containing wet and final dried wet granulates are also considered to be an aspect of the invention. For example, granulated mannitol could be used as a filler. It may also be desirable to granulate or pre-mix some portion of the formulation prior to final blending and compression.

Examples 1-3

Three substantially identical batches of wet granulate were produced from the components listed below in Table A. Note that the initial moisture content (calculated) was 36.8% based on the total weight of the materials used.

**Table A**

| Component | Batch Formula (kg/batch) |
|---|---|
| Desloratadine, Micronized | 38.0 |
| Microcrystalline Cellulose | 152.0 |
| Mannitol | 38.0 |
| Pregelatinized Starch | 14.4 |
| Sodium Starch Glycolate | 7.602 |
| Purified Water | 145.6 |

A 9% starch paste was prepared by mixing the pregelatinized starch in purified water with heating and mixing. This material was charged to a high shear granulator along with the remaining materials. The granulator used was a Wet High-Shear Granulater, Vertical, Bottom Driven (Fluid Air Pharm X 1250). Thereafter the wet granulate was dried in a fluid bed dryer (Fluid Air MagnaCoater) to obtain the partially dried wet granulate. The loss on drying was between 18% and 25%. The material was then milled in an impact mill (a conventional hammer mill/screening mill) with a rotating impeller and specifically a Fluid Air Granumill. After wet milling was completed, the partially dried milled wet granulate was returned to the same fluid bed dryer for the final drying step. Final measure of loss on drying was less than or equal to 1.8%. In this particular instance, the materials were screened after the second drying step and anything having a particle size sufficient to be retained on a 35 mesh screen (approximately 500 microns) was subsequently dry milled and mixed with the granulate whose particle size was less than 35 mesh. Sieving was done using a vibrator/shaker separator manufactured by Sweco. A more detailed description of the individual process parameters are described below.

1. Prepare the starch paste by adding pregelatinized starch to water while stirring at 60 ± 5°C.

2. Mix the desloratadine, microcrystalline cellulose, mannitol and sodium starch glycolate in a high-shear mixer for 5 minutes.

3. Slowly add the starch paste to the powder mixture while mixing at an impeller speed of 70±10 rpm.

4. Continue mixing at an impeller speed of 80±10 rpm until the granulation endpoint (torque of 2150 ft-lbs) is reached.

5. Partially dry the produced granules in the fluid bed dryer at an inlet temperature of 75 ± 5°C, until the LOD is 18-25% (i.e. 32.1-51.1% reduction in moisture content from theoretical initial moisture content).

6. Pass the partially-dried granules through a Granumill fitted with a 0.020" screen, and an impeller speed of 2500-3000 rpm. While milling, the milled granules are vacuum transferred back into the fluid bed using the following fluid bed settings: Vacuum Load Valve setting: 35%, Vacuum Transfer Air Flow setting: 0%, and Blower Load set point: 105 hp.

7. Dry the partially-dried-milled granules in the fluid bed dryer at an inlet temperature of 75 ± 5°C, until the LOD is ≤ 1.8% (i.e. at least a 95.1% reduction in moisture content from theoretical initial moisture content).

8. Sieve the dry granules through a #35 mesh screen using a Sweco Sifter or equivalent equipment.

9. Mill the granules that did not pass through the #35 mesh screen in the Granumill or equivalent equipment.

10. Combine the milled granules with the granules that passed through the #35 mesh screen for subsequent coating.

The moisture level determination is measured using a Mettler Toledo HR-73 Moisture Analyzer using the following settings:

| | |
|---|---|
| Drying Program.: | Standard Drying |
| Drying Temp.: | 105°C |
| Switch off Criteria: | Loss< 1mg/50 sec. |
| Drying Time: | --------- |
| Display Mode Result: | MC (Moisture Content) |
| Print Interval: | 30 Sec. |
| Target Weight: | 4.000 g |

The three batches produced utilizing the above process produced granules having the following properties:

| | Example 1 | Example 2 | Example 3 |
|---|---|---|---|
| Mean particle size (microns) | 221 | 238 | 253 |
| Amount of particles < 125 microns (%w/w) | 20.3 | 13.4 | 6.5 |
| Amount of particles within 50% of the mean particle size (%w/w) | 66.6 | 71.3 | 90.5 |

Attempts to wet mill several batches utilizing similar wet granules without the partial drying step were unsuccessful due to blinding of the mill screen. A wide range of screen sizes, ranging from 0.094" to 1.0" , were tried; all were blinded by the wet material and the milling process was unsuccessful. The partial drying step allowed for the successful milling of the partially dried material through screens as small as 0.020".

Examples 4-11

Eight batches of fexofenadine containing wet granulate was produced as described below using the formulation substantially shown in Table B. The initial moisture content of the batches (calculated) was 23.5%.

**Table B**

| Component | Batch Formula (kg/batch) |
|---|---|
| Fexofenadine HCl | 51.0 |
| Microcrystalline Cellulose | 93.5 |
| Sodium Starch Glycolate | 17.0 |
| Povidone | 8.5 |
| Alcohol, SDA-3A, Anhydrous | 40.0 |

The general process used for production of the fexofenadine hydrochloride containing granulate is similar to that in Examples 1-3. In particular, the high shear granulator, partial drying, milling, and sieving steps were undertaken using the same equipment as previously specified in those examples. The fexofenadine HC1, povidone microcrystalline cellulose and sodium starch glycolate were weighed into the high shear granulator and separately the alcohol was weighed and added. Granulation proceeded as described below and once granulation was complete, the material was partially dried in the fluidized bed to produce the partially dried wet granulate. After the initial drying step, the resulting partially dried wet granulate was screened with a 30 and 40 mesh screen. Any particles that were retained on the 30 or 40 mesh screens were milled as described herein through a 0.020 inch screen. The materials that passed through the 40-mesh screen were not separately milled. Thereafter, both the milled oversized granules and the unmilled granules are recombined in the fluid air bed for final drying.

More specifically, the manufacturing process was as follows:

Mix the fexofenadine HCl, microcrystalline cellulose, sodium starch glycolate and povidone in a high-shear mixer for 5 minutes.

Add the alcohol, SDA-3A, anhydrous to the powder mixture while mixing at an impeller speed of 50 rpm.

Continue mixing at an impeller speed of 50 rpm for 5 minutes.

Partially dry the produced granules in the fluid bed dryer at an inlet temperature of 50 ± 5°C, until the LOD is 5-8% (i.e. 66.0-78.8% reduction in moisture content from theoretical initial moisture content).

Screen the partially dried granules using a Sweco Separator equipped with #30 and #40 mesh screens. The granulation will be separated into the following sieve fractions: > 30 mesh, <30/>40 mesh and < 40 mesh. The <40 mesh partially-dried granules are retained until the final drying process. The > 30 and <30/>40 mesh partially-dried granules proceed to the milling process.

Pass the > 30 mesh and <30/>40 mesh partially-dried granules through a Granumill with a 0.020" screen, and an impeller speed of 1000 rpm. While milling, the milled granules are vacuum transferred back into the fluid bed using the following fluid bed settings: Vacuum Load Valve setting: 35%, Vacuum Transfer Air Flow setting: 0%, and Blower Load set point: 105 hp.

Once the milling has been completed, the < 40 mesh partially-dried granules are vacuum loaded into the fluid bed and are combined with the milled granules.

Dry the partially-dried-milled and un-milled granules in the fluid bed dryer at an inlet temperature of 50 ± 5°C, until the LOD is ≤ 3.0% (i.e. at least a 87.2% reduction in moisture content from theoretical initial moisture content).

The finished granules are discharged and retained for subsequent coating.

The moisture level determination is measured using a Mettler Toledo HR-73 Moisture Analyzer using the following settings:

| | |
|---|---|
| Drying Program.: | Standard Drying |
| Drying Temp.: | 105°C |
| Switch off Criteria: | Loss< 1mg/50 sec. |
| Drying Time: | ---------- |
| Display Mode Result: | MC (Moisture Content) |
| Print Interval: | 30 Sec. |
| Target Weight: | 4.000 g |

The eight batches utilizing the above process produced granules having the following properties:

| | Ex.4 | Ex.5 | Ex.6 | Ex.7 | Ex.8 | Ex.9 | Ex.10 | Ex.11 |
|---|---|---|---|---|---|---|---|---|
| Mean particle size (microns) | 217 | 222 | 226 | 279 | 218 | 226 | 213 | 204 |
| Amount of particles < 125 microns (%w/w) | 21.8 | 32.0 | 22.6 | 11.0 | 26.7 | 28.3 | 31.0 | 36.2 |
| Amount of particles within 50% of the mean particle size (%w/w) | 71.4 | 63.2 | 70.0 | 65.8 | 67.6 | 63.1 | 68.4 | 64.9 |

Attempts to wet mill several batches utilizing similar wet granules without the partial drying step were unsuccessful due to blinding of the mill screen and/or unacceptable particle size distribution. A 0.040" screen was used and caused immediate blinding of the mill screen. A 0.078" screen was used and caused approximately half (49%) of the granulation to be larger than 40 mesh in particle size and only 19% to be within the desired particle size range for further processing. The partial drying step allowed for the successful milling of the partially dried material through screens as small as 0.020".

### INDUSTRIAL APPLICABILITY

This present invention is relevant to the pharmaceutical industry and specifically to the manufacture of dosage forms and medicines.

### Embodiments

1. A method of producing a wet granulate having a desirable particle size distribution comprising the steps of: drying a wet granulate having an initial moisture content comprising at least one pharmaceutically active ingredient to a predetermined first relative moisture content to form a partially dried wet granulate; milling said partially dried wet granulate to obtain a milled partially dried wet granulate predetermined particle size distribution; and drying said milled partially dried wet granulate to produce a final dried wet granulate, wherein said predetermined first relative moisture content is at least about 30% less than the initial moisture content of said wet granulate.
2. The method of item 1, wherein said final dried wet granulate has an average particle size of between about 150 and about 600 microns and no more than about 40% of the particles by weight have a particle size of 105 microns.
3. The method of item 2, wherein said step of milling said partially dried wet granulate is accomplished using a screen which is 20 and 140 mesh.
4. The method as in any one of items 1, 2 and 3, wherein said predetermined first relative moisture content is between about 30% and about 85% less than said initial moisture content of said wet granulate.
5. The method of item 4, wherein said predetermined first relative moisture content is between about 40% and about 80% less than said initial moisture content of said wet granulate.
6. The method of item 5, wherein said predetermined first relative moisture content is between about 50% and about 80% less than said initial moisture content of said wet granulate.
7. The method of item 6, wherein said predetermined first relative moisture content is between about 60% and about 75% less than said initial moisture content of said wet granulate.
8. The method of item 4, wherein said final dried wet granulate has an average particle size of between about 175 and about 600 microns and no more than about 35% of the particles by weight have a particle size of 105 microns.
9. The method of item 8, wherein said final dried wet granulate has an average particle size of between about 200 and about 600 microns and no more than about 35% of the particles by weight have a particle size of 105 microns.
10. A method of producing a wet granulate having a desirable particle size distribution comprising the steps of: drying a wet granulate comprising at least one pharmaceutically active ingredient to a predetermined first relative moisture content to form a partially dried wet granulate, wherein said predetermined first relative moisture content is at least about 30% less than the initial moisture content of said wet granulate; milling said partially dried wet granulate using a screen which is between about 20 and about 140 mesh to obtain a predetermined particle size distribution; and drying said milled partially dried wet granulate to produce a final dried wet granulate having an average particle size of between about 175 and about 600 microns and no more than about 35% of the particles by weight have a particle size of 105 microns.
11. The method of item 10, wherein said predetermined first relative moisture content is between about 40% and about 80% less than said initial moisture content of said wet granulate.
12. The method of item 11, wherein said predetermined first relative moisture content is between about 50% and about 80% less than said initial moisture content of said wet granulate.
13. The method of item 12, wherein said final dried wet granulate has an average particle size of between about 200 and about 600 microns and no more than about 30% of the particles by weight have a particle size of 105 microns.
14. The method of item 10, wherein said wet granulate further comprises at least one excipient.
15. The method of item 10, wherein said at least one pharmaceutically active ingredient is fexofenadine, desloratadine, fentanyl, tramadol, modafinil, armodafinil, clozapine, azithromycin or oxycodone.
16. The method of item 10, further comprising coating said final dried wet granulate.
17. The method of item 16, wherein said coating is a taste masking coating.
18. A final dried wet granulate produced according to the steps of item 1.
19. A final dried wet granulate produced according to the steps of item 4.
20. A final dried wet granulate produced according to the steps of item 10.
21. A final dried wet granulate produced according to the steps of item 17.
22. A dried wet granulate comprising: at least one pharmaceutically active ingredient granulate and at least one excipient, said dried wet granulate having an average particle size of between about 150 and about 600 microns and no more than about 40% of the particles by weight have a particle size of 105 microns.
23. The dried wet granulate of item 22, further comprising at least one coating.
24. The dried wet granulate of item 23, wherein said coating is a taste masking coating.
25. A compressed tablet comprising: dried wet granulate comprising at least one pharmaceutically active ingredient granulate and at least one excipient, said dried wet granulate having an average particle size of between about 150 and about 600 microns and no more than about 40% of the particles by weight have a particle size of 105 microns; and at least one excipient, which have been compressed into a tablet.
26. The method of item 25, wherein said at least one pharmaceutically active ingredient is fexofenadine, desloratadine, fentanyl, tramadol, modafinil, armodafinil, clozapine, azithromycin or oxycodone.
27. The method of item 26, wherein said at least one pharmaceutically active ingredient is fexofenadine, desloratadine, or fentanyl.

## Claims

1. A granulate comprising a pharmaceutically active ingredient and an excipient, wherein the granulate is obtainable by:
drying a wet granulate having an initial moisture content to a predetermined first relative moisture content that is between about 30% and about 85% less than said initial moisture content to form a partially dried wet granulate, wherein the wet granulate comprises the pharmaceutically active ingredient and the excipient;
milling said partially dried granulate to obtain a milled partially dried granulate having a predetermined particle size distribution; and,
drying said milled partially dried granulate to produce said granulate,
wherein said pharmaceutically active ingredient is oxycodone.

2. The granulate according to claim 1 wherein said dried granulate comprises spherinized particles.

3. The granulate according to claim 1 or 2 wherein the particles of said dried granulate are coated with a taste masking coating.

4. The granulate according to claim 1 or 2 wherein the particles of said dried granulate are coated with a controlled release coating.

5. The granulate according to claim 1 wherein said excipient is an effervescent material.

6. The granulate according to claim 1 wherein said excipient is a pH adjusting substance.

7. The granulate according to claim 1 wherein said excipient is a filler.

8. The granulate according to claim 1 having an average particle size of between about 150 and 600 microns and wherein no more than about 40% of the particles by weight have a particle size of 105 microns or less.

9. The granulate according to claim 8, wherein no more than about 30% of the particles by weight have a particle size of 105 microns or less.

10. The granulate according to any one of claims 1 to 9, wherein the average particle size is less than about 500 microns.

11. The granulate according to any one of claims 1 to 9, wherein the average particle size measurements based on the final dried granulate range from between 220 microns and 250 microns or wherein the average particle size measurement based on the final dried granulate is about 220 microns or about 250 microns.

12. The granulate according to claim 10 or 11, wherein not less than about 60% of the particles (w/w) fall within 50% of said average particle size.

13. The granulate according to claim 1 wherein said milling is performed using a 20 to 140 mesh screen.

14. A tablet comprising a granulate according to any one of claims 1 to 13.

## Patentansprüche

1. Granulat, umfassend einen pharmazeutischen Wirkstoff und einen Hilfsstoff, wobei das Granulat dadurch erhältlich ist, dass man:
ein feuchtes Granulat mit einem anfänglichen Feuchtigkeitsgehalt bis zu einem vorbestimmten ersten relativen Feuchtigkeitsgehalt, der zwischen etwa 30% und etwa 85% kleiner als der anfängliche Feuchtigkeitsgehalt ist, trocknet, wobei man ein teilweise getrocknetes feuchtes Granulat erhält,
wobei das feuchte Granulat den pharmazeutischen Wirkstoff und den Hilfsstoff umfasst;
das teilweise getrocknete Granulat mahlt, wobei man ein gemahlenes teilweise getrocknetes Granulat mit einer vorbestimmten Teilchengrößenverteilung erhält; und
das gemahlene teilweise getrocknete Granulat trocknet, wobai man das Granulat erhält;
wobei es sich bei dem pharmazeutischen Wirkstoff um Oxycodon handelt.

2. Granulat nach Anspruch 1, wobei das getrocknete Granulat sphäronisierte Teilchen umfasst.

3. Granulat nach Anspruch 1 oder 2, wobei die Teilchen des getrockneten Granulats mit einem geschmacksmaskierenden Überzug beschichtet sind.

4. Granulat nach Anspruch 1 oder 2, wobei die Teilchen des getrockneten Granulats mit einem Überzug zur kontrollierten Wirkstofffreigabe beschichtet sind.

5. Granulat nach Anspruch 1, wobei es sich bei dem Hilfsstoff um ein Brausematerial handelt.

6. Granulat nach Anspruch 1, wobei es sich bei dem Hilfsstoff um eine den pH-Wert regulierende Substanz handelt.

7. Granulat nach Anspruch 1, wobei es sich bei dem Hilfsstoff um einen Füllstoff handelt.

8. Granulat nach Anspruch 1 mit einer durchschnittlichen Teilchengröße zwischen etwa 150 und 600 Mikron, wobei höchstens etwa 40 Gew.-% der Teilchen eine Teilchengröße von 105 Mikron oder weniger aufweisen.

9. Granulat nach Anspruch 8, wobei höchstens etwa 30 Gew.-% der Teilchen eine Teilchengröße von 105 Mikron oder weniger aufweisen.

10. Granulat nach einem der Ansprüche 1 bis 9, wobei die durchschnittliche Teilchengröße weniger als etwa 500 Mikron beträgt.

11. Granulat nach einem der Ansprüche 1 bis 9, wobei die Messungen der durchschnittlichen Teilchengröße auf Basis des fertigen getrockneten Granulats im Bereich zwischen 220 Mikron und 250 Mikron liegen oder die Messung der durchschnittlichen Teilchengröße auf Basis des fertigen getrockneten Granulats etwa 220 Mikron oder etwa 250 Mikron beträgt.

12. Granulat nach Anspruch 10 oder 11, wobei mindestens etwa 60% der Teilchen (Gew./Gew.) innerhalb von 50% der durchschnittlichen Teilchengröße fallen.

13. Granulat nach Anspruch 1, wobei das Mahlen unter Verwendung eines 20- bis 140-mesh-Siebs erfolgt.

14. Tablette, umfassend ein Granulat nach einem der Ansprüche 1 bis 13.

## Revendications

1. Granules comprenant un principe actif pharmaceutique et un excipient, les granules pouvant être obtenus par :
séchage des granules humides comportant une teneur initiale en humidité pour obtenir une première teneur prédéterminée en humidité relative environ 30 % à environ 85 % inférieure à ladite teneur en humidité initiale pour former des granules humides partiellement séchés, les granules humides comprenant le principe actif pharmaceutique et l'excipient ;
broyage desdits granules partiellement séchés pour obtenir des granules partiellement séchés broyés de distribution granulométrique prédéterminée : et,
séchage desdits granules partiellement séchés broyés pour obtenir lesdits granules,
ledit principe actif pharmaceutique étant l'oxycodone.

2. Granules conformes à la revendication 1, lesdits granules séchés comprenant des particules sphérifiées.

3. Granules conformes à la revendication 1 ou 2, où les particules desdits granules séchés sont revêtues d'un enrobage masquant le goût.

4. Granules conformes à la revendication 1 ou 2, où les particules desdits granules séchés sont revêtues d'un enrobage de libération contrôlée.

5. Granules conformes à la revendication 1, où ledit excipient est une matière effervescente.

6. Granules conformes à la revendication 1, où ledit excipient est une substance d'ajustement du pH.

7. Granules conformes à la revendication 1, où ledit excipient est une charge.

8. Granules conformes à la revendication 1, de granulométrie moyenne comprise entre environ 150 et 600 microns, et où jusqu'à environ 40 % en masse des particules présentent une granulométrie de 105 microns ou moins.

9. Granules conformes à la revendication 8, où jusqu'à environ 30 % des particules présentent une granulométrie de 105 microns ou moins.

10. Granules conformes à l'une quelconque des revendications 1 à 9, où la granulométrie moyenne est inférieure à environ 500 microns.

11. Granules conformes à l'une quelconque des revendications 1 à 9, où la granulométrie moyenne basée sur les granules séchés finaux est comprise entre 220 microns et 250 microns, ou où la granulométrie moyenne basée sur les granules séchés finaux est d'environ 220 microns ou d'environ 250 microns.

12. Granules conformes à la revendication 10 ou 11, où jusqu'à environ 60 % en masse des particules mesurent moins de 50 % de ladite granulométrie moyenne.

13. Granules conformes à la revendication 1, où ledit broyage est mis en oeuvre sur un crible de maille 20 à 140.

14. Comprimé comprenant des granules conformes à l'une quelconque des revendications 1 à 13.
